# EUROPEAN PATENT APPLICATION

(11) **EP 1 371 642 A1**
(43) Date of publication of application: **17.12.2003**
(21) Application number: 03008401.6
(22) Date of filing: 11.04.2003
(51) Int. Cl.: C07C 403/24

(54) **Process for the production of canthaxanthin**

(30) Priority: 11.06.2002 DE 10225856
(71) Applicant: Degussa AG, 40474 Düsseldorf (DE)
(72) Inventor: Dübner, Frank, Dr., 61196 Friedberg (DE); Bilz, Jürgen, 63579 Freigericht (DE); Weckbecker, Christoph, Dr., 63584 Gründau (DE); Huthmacher, Klaus, Dr., 63517 Gelnhausen (DE)

(57) **Abstract**

The invention relates to a process for the production of canthaxanthin by oxidation of beta-carotene or carotinoids derived therefrom having conjugated double bonds.

## Description

### Field of the Invention

The invention provides a process for the production of canthaxanthin by oxidation of beta-carotene or carotinoids derived therefrom having conjugated double bonds.

### Background of the Invention

Canthaxanthin is one of many natural pigments and is found in a large number of animals and plants. It has long been widely used as a red dye both in aquaculture and poultry rearing.

Known processes for the formation of canthaxanthin from beta-carotene use either salts of chloric or bromic acid (DE 2534805), salts of periodic acid (DT 1793308) or manganese oxides (Lu, Yu; Kuang, Binghai; Zhang, Bin, *Fine Chemicals,* China, 2000, **17**, 74-76) as the oxidising agent. However, long reaction times or high excesses of oxidising agent are needed for complete conversion. The processes described are thus uneconomical and associated with safety concerns.

In example 1 of DT 1793308 beta-carotene is reacted with 4 equivalents of sodium periodate and 2 mol-% iodine in a two-phase mixture of chloroform and water. After a reaction time of two hours, canthaxanthin is obtained by precipitation at a yield of 33.7%. No details are given of the desired all-trans content.

Example 1 of DE 2534805 discloses the oxidation of beta-carotene with ten equivalents of sodium chlorate with the addition of catalytic quantities of iodine. The reaction is complete only after 36 hours (h) at room temperature and, after precipitation, yields 69.5% canthaxanthin with an all-trans content of 82%. In example 2, the yield can be increased to 78.5%, if no less than 30 equivalents of sodium chlorate are used. The use of catalysts other than iodine, such as e.g. potassium iodide or oxides of elements from Group Va, VIa or VIIa produced poorer results.

In EP-A-1059290 the reaction time is successfully reduced to 2 h with comparable yields by using the catalysts iodine and potassium iodide at the same time. However here too, 15 mol-equivalents of sodium chlorate, which is fire-promoting and tends towards explosiveness at high temperatures, are needed. At the end of the reaction a mixture is obtained that contains 76% canthaxanthin with an all-trans content of 52%.

### Object of the Invention

In view of the prior art, it would be desirable to provide a process that requires no fire-promoting chemicals and can nevertheless be carried out in short reaction times.

### Summary of the Invention

The invention is a process for the production of canthaxanthin, in which direct oxidation of beta-carotene, or one of the precursors of canthaxanthin described above, to canthaxanthin can be carried out with commercial salts of hypohalogen acids or released hypohalogen acids, such as for example HOCl HOI or HOBr, and their decomposition products, with complete conversion and good selectivity.

In particular, the invention is a process for the production of canthaxanthin by oxidation of beta-carotene or other oxidation intermediates, in the presence of
a) an oxidizing agent,
b) a catalyst, and
c) an inert, organic solvent,
characterized in that a salt of hypohalogen acids or the hypohalogen acids released "in situ" by acidification of the aqueous solutions of these salts, and the decomposition products thereof, are used as the oxidizing agent.

### Detailed Description of the Invention

The process according to the invention for the production of canthaxanthin comprises the oxidation of beta-carotene, retro-dehydrocarotene, echinenone, isozeaxanthin, isocryptoxanthin, 4'-hydroxyechinenone or other oxidation intermediates with salts of hypohalogen acids or released hypohalogen acids, such as for example, HOCl or HOBr, and their decomposition products, in a pH of 4-8, in the presence of halogens, such as chlorine, bromine or iodine or of oxides or oxo acids of the elements of Groups Va, VIa, or VIIa, or of oxides of Group VIII of the periodic system, or the salts thereof, and an organic solvent inert towards the reaction partners at low temperatures.

This process is characterized in that free halogens chlorine, bromine or iodine, or the alkali, earth alkali or ammonium salts of the corresponding hydrohalic acids or oxides of the oxo acids of selenium or of one of the elements of Groups Va, VIa, or VIIa, or a salt of these oxo acids or of oxides of the elements of Group VIII are used as the catalyst; in the presence of an inert organic solvent; that the reaction takes place at low temperatures, and that either a salt of hypochlorous acid, hypobromous acid or hypoiodous acid or the released acids HOCl, HOBr or HOI and their decomposition products are themselves used as the oxidizing agent and the reaction takes place at a temperature in the range -10°C to 70°C and echinenone or other carotinoids with a lower oxidation number than canthaxanthin is also used as the starting material.

Substantial advantages of the process according to the invention in comparison with the processes known hitherto are the use of chlorine bleach solution (NaOCl solution in water), which is inexpensive and preferable from the point of view of safety, the use of only 4 equivalents of oxidizing agent and the very short reaction times. The process is thus more economical than all methods previously disclosed.

The starting materials are oxidized in the form of solutions. Solutions of 1 to 40 g of the carotinoid to be oxidized in 1 liter of an organic solvent inert towards the reaction partners and not miscible with water, are preferred.

Possible inert, organic solvents are chlorinated aliphatic hydrocarbons, for example chloroform, dichloromethane, 1,1-dichloroethane, 1,2-dichloroethane, 1,1,2-trichloroethane, 1,1,2,2-tetrachloroethane, 1,2-dichloroethylene, 1,1,2-trichloroethylene, aromatic hydrocarbons, for example benzene, toluene, nitrobenzene or chlorobenzene, dialkylethers, for example diethylether, di-n-propylether or carbon disulfide. Chloroform and dichloromethane are particularly suitable solvents.

Possible oxidizing agents are salts of hypochlorous acid or hypobromous acid, in particular the earth alkali-, alkali- or ammonium salts or the free acids produced "in situ" and their decomposition products, which form when acidifying the salt solutions. Examples of oxidizing agents are sodium hypochlorite, potassium hypochlorite, sodium hypobromite, potassium hypobromite, sodium hypoiodite, or potassium hypoiodite. Preferred oxidizing agents are sodium hypochlorite and sodium hypobromite, sodium hypochlorite being preferred in particular.

Decomposition products are for example free halogen acids, such as chloric, bromic or iodic acids at a pH below 7, or their salts, such as chlorates, bromates or iodates at a pH above 7.

The oxidizing agents are usefully added to the reaction mixture in the form of aqueous solutions at a concentration of 1 to 15 wt.%. The molar ratio of oxidizing agent to starting substance is in the range 1:1 to 10:1, preferably in the range 1:1 to 5:1 mol. A more than 10-fold molar excess of oxidizing agent leads to losses in yield.

Oxidation is catalyzed by the halogens chlorine, bromine or iodine, or by oxides or oxo acids of selenium, or by the oxides or oxo acids of elements of groups Va, VIa, or VIIa, or oxides of group VIII of the periodic system, or their salts.

Suitable catalysts are, for example, selenium dioxide, selenious acid or its salts, selenic acid or its salts, vanadium pentoxide, vanadates, polyvanadic acids or their salts, heteropolyacids of vanadium, in particular with the elements tungsten, molybdenum and phosphorus, or their salts, molybdenum trioxide, molybdates, in particular ammonium molybdate, polymolybdates, heteropolyacids of molybdenum, in particular with the elements vanadium or phosphorus, tungsten trioxide, tungstates, polytungstic acids or their salts, heteropolyacids of tungsten, in particular with the elements vanadium and phosphorus or their salts, manganese dioxide, nickel oxide, osmium tetroxide, or mixtures thereof. Preferred catalysts are the free halogens chlorine, bromine, iodine and the alkali, earth alkali or ammonium salts of the corresponding hydrohalic acids, such as for example sodium chloride, -bromide or -iodide, potassium chloride, -bromide or -iodide, or ammonium chloride, -bromide or -iodide, and osmium tetroxide. Elemental iodine and potassium iodide or a mixture of both are particularly suitable as a catalyst.

The catalyst is added in pure form or in solution, e.g. in the solvent, that was used to dissolve the carotinoid to be oxidized, or in water. Equally, the catalyst can be formed "in situ". The quantity of catalyst is usefully 0.1 to 10 wt.%, preferably 1 to 5 wt.%, in relation to the carotinoid to be oxidized.

As a result of the thermal sensitivity of the substances, oxidation is carried out at low temperatures, such as for example -10°C to 70°C, preferably 0°C to 10°C.

Conversion takes place in a pH range, starting from the neutral range and extending into the slightly acid pH value. The reaction preferably takes place at a pH in the range 4 to 8. To set the desired pH value, acids, for example sulfuric acid, hydrochloric acid, acetic acid, or buffer mixtures, are used.

Depending on the conditions selected, the reaction lasts for 0.1 to 10 hours, preferably 0.1 to 5 hours. In a particularly advantageous embodiment, 1 hour is needed to obtain the optimum yield of canthaxanthin.

To prevent canthaxanthin from being oxidized by the effects of atmospheric oxygen, the reaction is carried out in an inert gas atmosphere. Inert gases that are suitable under the reaction conditions are argon, neon, helium, carbon dioxide, in particular nitrogen.

The present invention is explained in more detail below with the aid of embodiment examples.

### Example 1

A solution of 55 mg iodine in 30 ml dichloromethane is added to 4 g beta-carotene in 270 ml dichloromethane in a nitrogen atmosphere at 0-5°C. 20 ml chlorine bleach solution (NaOCl, ca 12% active chlorine content) is then added drop by drop at this temperature within 30 minutes. Before this, the chlorine bleach solution is slightly acidified (pH 6) with 1 N H₂SO₄ and is used within 10 minutes. After addition, the mixture is stirred for a further 30 minutes at 0°C. During this time, the organic phase darkens increasingly. The mixture is diluted with water and the phases are separated. The organic phase is washed with water, dried over sodium sulfate and the solvent is removed in a vacuum. 4.8 g of the raw product with a canthaxanthin content of 80 surface percent is obtained. The all-trans content of the canthaxanthin measured by HPLC is 71%. For isomerization, 25 ml acetone and 40 mg iodine are added to the residue, which is refluxed until a z-isomer signal no longer occurs in HPLC.

The canthaxanthin can be separated out of the reaction mixture by the usual methods for the separation of constituents of a mixture. For example, the solvent can be removed by distillation at reduced pressure and the canthaxanthin can be isolated by recrystallization.

### Example 2

A solution of 40 mg sodium iodide in 10 ml water is added to 4 g beta-carotene in 270 ml dichloromethane in a nitrogen atmosphere at 0-5°C. 20 ml chlorine bleach solution (NaOCl, ca 12% active chlorine content) is then added drop by drop at this temperature within 30 minutes. Before this, the chlorine bleach solution is slightly acidified (pH 6) with 1 N H₂SO₄ and used within 10 minutes. After addition, the mixture is stirred for a further 30 minutes at 0°C. During this time, the color of the organic phase darkens increasingly. The mixture is diluted with water and the phases are separated. The organic phase is washed with water, dried over sodium sulfate and the solvent is removed in a vacuum. 4.9 g of the raw product with a canthaxanthin content of 72 surface percent is obtained. The all-trans content of the canthaxanthin measured by HPLC is 65%. For isomerization, 25 ml acetone and 40 mg iodine are added to the residue, which is refluxed until a z-isomer signal no longer occurs in HPLC.

| Analysis conditions: | |
|---|---|
| Column | Inertsil C-8 5µ 250 x 4.6, 40°C, 1.0 ml/min |
| Solvent | Water/Methanol/THF from 10/1/9 to 9/2/29 |
| Detector | UV/VIS 450 nm |

## Claims

1. Process for the production of canthaxanthin by oxidation of beta-carotene or other oxidation intermediates, in the presence of
a) an oxidizing agent,
b) a catalyst, and
c) an inert, organic solvent
wherein a salt of hypohalogen acids or the hypohalogen acids released "in situ" by the acidification of the aqueous solutions of these salts and the decomposition products thereof are used as the oxidizing agent.

2. Process according to claim 1, wherein the oxidizing agent is an earth alkali-, alkali- or ammonium salt of hypochlorous acid, hypobromous acid or hypoiodous acid, or the free acids produced "in situ" by the acidification of the aqueous solutions of these salts and the decomposition products thereof.

3. Process according to claim 1 or 2, wherein the oxidizing agent is produced "in situ" from sodium hypochlorite.

4. Process according to claim 1, wherein the oxidation intermediates are retro-dehydrocarotene, echinenone, isozeaxanthin, isocryptoxanthin, or 4'-hydroxyechinenone.

5. Process according to claim 1, wherein it is carried out at temperatures of -10°C to 70°C.

6. Process according to claim 5, wherein the temperature is 0°C to 10°C.

7. Process according to claim 1, wherein the inert, organic solvent is chlorinated aliphatic hydrocarbons, aromatic hydrocarbons, dialkyl ethers or carbon disulfide.

8. Process according to claim 7, wherein the inert, organic solvent is chloroform, dichloromethane, 1,1-dichloroethane, 1,2-dichloroethane, 1,1,2-trichloroethane, 1,1,2,2-tetrachloroethane, 1,2-dichloroethylene, 1,1,2-trichloroethylene, benzene, toluene, nitrobenzene or chlorobenzene, diethylether, di-n-propylether.

9. Process according to claim 8, wherein the solvent is chloroform or dichloromethane.

10. Process according to claim 1, wherein the catalyst is a halogen, an oxide or an oxo acid of the elements of Groups Va, VIa, or VIIa, or an oxide of Group VIII of the periodic system, or the salts thereof.

11. Process according to claim 10, wherein the catalyst is elemental chlorine, bromine or iodine or an alkali, earth alkali or ammonium salt of the corresponding hydrohalic acid, a mixture of these or osmium tetroxide.

12. Process according to claim 10 or 11, wherein the catalyst is elemental iodine.

13. Process according to claim 1, wherein the molar ratio of oxidizing agent to starting substance is 1:1 to 10:1 mol.

14. Process according to claim 13, wherein the molar ratio of oxidizing agent to starting substance is 1:1 to 5:1 mol.

15. Process according to claim 1, wherein oxidation is carried out at a pH of 4 to 8.

16. Process according to claim 1, wherein the reaction time is 0.1 to 10 hours.

17. Process according to claim 1 or 16, wherein the reaction time is 1 hour.
